(19) Europäisches Patentamt
European Patent Office
Office européen des brevets

(11) **EP 3 383 350 B1**

(12) **EUROPEAN PATENT SPECIFICATION**

(45) Date of publication and mention
of the grant of the patent:
**13.03.2019 Bulletin 2019/11**

(21) Application number: **16787884.2**

(22) Date of filing: **28.10.2016**

(51) Int Cl.:
*A61K 8/02* (2006.01)    *A61K 8/19* (2006.01)
*A61K 8/26* (2006.01)    *A61K 8/67* (2006.01)
*A61K 8/34* (2006.01)    *A61K 8/49* (2006.01)
*A61K 8/35* (2006.01)    *A61K 8/36* (2006.01)
*A61K 8/365* (2006.01)    *A61K 8/81* (2006.01)
*A61Q 19/08* (2006.01)    *A61Q 19/02* (2006.01)

(86) International application number:
**PCT/EP2016/076035**

(87) International publication number:
**WO 2017/092937 (08.06.2017 Gazette 2017/23)**

(54) **A COSMETIC MASK**

KOSMETISCHE MASKE

MASQUE COSMÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2015 PCT/CN2015/096042
18.01.2016 EP 16151688**

(43) Date of publication of application:
**10.10.2018 Bulletin 2018/41**

(73) Proprietors:
• **Unilever NV
3013 AL Rotterdam (NL)**
Designated Contracting States:
**AL AT BE BG CH CZ DE DK EE ES FI FR GR HR
HU IS IT LI LT LU LV MC MK NL NO PL PT RO RS
SE SI SK SM TR**
• **Unilever PLC
London, Greater London EC4Y 0DY (GB)**
Designated Contracting States:
**CY GB IE MT**

(72) Inventors:
• **AO, Mingqi
Shanghai 200335 (CN)**
• **EKANI NKODO, Axel, Herve
Wirral Merseyside CH63 3JW (GB)**
• **GHATLIA, Naresh, Dhirajlal
Shanghai 200335 (CN)**
• **LI, Hangsheng
Shanghai 200125 (CN)**
• **YUAN, Caigen
Shanghai 200335 (CN)**
• **WANG, Wei
Shanghai 200335 (CN)**

(74) Representative: **Corsten, Michael Allan
Unilever N.V.
Unilever Patent Group
Olivier van Noortlaan 120
3133 AT Vlaardingen (NL)**

(56) References cited:
**EP-A1- 2 942 048    WO-A2-2008/010167**

Remarks:
The file contains technical information submitted after
the application was filed and not included in this
specification

**Description**

**Field of the invention**

The invention relates to a cosmetic mask. The invention more particularly relates to a cosmetic mask which can be applied to the face or any other topical surface of the body for providing both the benefits of a skin care active that delivers long-term benefits as well as instant lightening benefits without the disadvantages of unnatural whiteness that is often associated with such instant lightening products.

**Background of the invention**

Consumers look to cosmetic products for delivering various benefits to skin e.g. skin lightening, anti-aging, anti-acne and various other benefits. Several of the agents known and used to deliver such benefits work by modifying the biological function at the surface (or *stratum corneum*) level of the skin. Such actives are also known as biological actives or bioactives and are different from inert (and often inorganic) actives that interact with the incident light to deliver an optical benefit to the skin. It has been observed that bioactives are often difficult to formulate in skin care composition as they may interact with other ingredients present in the formulation to cause certain problems like stability, colour, reduced efficacy, among other problems. Thus, when there is a need to deliver more than one benefit to the skin, the scientist faces a challenge in doing so.

In the present invention, the inventors seek to deliver the varied benefits of the broad area of anti-aging, which is visible as reduction in wrinkles and skin discontinuities while ensuring skin lightening. Skin lightening includes instant lightening benefits as well as long term reduction in the baseline colour of the skin. Skin lightening is evidenced with a general reduction in the overall colour tone of the skin as well as age spots, hyperpigmentation and age related discolouration. Thus anti-aging and skin lightening are related and overlapping biological phenomena which cannot be linearly addressed.

The exposure of the skin to the sun and other environmental irritants causes it to darken, often in an uneven and blotchy manner as well as cause age-related spotting and wrinkling. To tackle this problem the present inventors wish to incorporate bioactives that are known to ameliorate such skin conditions while also provide instant lightening benefits.

As has been mentioned above, formulating such bioactives in a conventional gel, cream, lotion or emulsion based composition is often a challenge as there is likelihood of the ingredients interacting with each other thereby affecting the stability and other desirable characteristics like colour of the formulation. The present inventors realized through extensive experimentation that the objectives of the present invention could be better delivered though the class of cosmetic products called face mask. Such products consist of a paper (or similar water insoluble substrate) into which the desired actives with minimal amount of other adjuvants are impregnated and the mask is then applied on to the face for a short or long period of time (say a few minutes to several hours e.g. overnight application). Thereafter, the mask is removed and the face may then be rinsed with water, if required. Such products have been claimed to give benefits against problems like acne by removing the dead skin cells on the surface and by removing unwanted oil and other irritants. Such products have also been claimed to moisturize the skin through use of high levels of humectants and other moisturizing agents. The inventors realized that an additional advantage of delivering the desired actives of the present invention through a face mask was that the actives could be impregnated into the water insoluble substrate at close to room temperature and does not require high temperature mixing that is often needed in order to prepare stable emulsions. This advantage not only manifests in lower cost but also ensures a more stable composition especially when the actives may be temperature sensitive.

The present inventors wish to deliver the benefits of skin lightening, anti-aging and other benefits by including actives like retinol, retinyl esters, resorcinol, vitamin B3, allantoin, ubiquinone, conjugated linoleic acid, 12-hydroxystearic acid or derivatives thereof; along with instant whitening actives through the above vehicle of a face mask. They found that when conventional instant skin lightening actives like titanium dioxide are used there are problems of stability leading to yellowing of the substrate and this can be solved by including certain other actives like certain polymeric beads, boron nitride or mica. Further, they surprisingly found that the benefits can be delivered in an enhanced fashion by including film forming polymers.

US2004219124 (Bioderm) discloses a cosmetic or pharmaceutical mask composition suitable for facial, hair, skin, or body application comprising: (i) At least one biopolymer, polymer, or polyvalent metal complexing composition as one component of the ion-pair, which forms the matrix of mask composition (Part I), from about 0.1% to about 50%, and (ii) at least one divalent or trivalent metal cation as the other component of the ion-pair (Part II), which causes the cross-linking of the first component of the ion-pair composition (Part I), from about 0.1% to about 50% and (iii) at least one skin, hair, or body beneficial cosmetic or pharmaceutical composition, from about 0. 0001% to about 25%, and (iv) a cosmetically or pharmaceutically acceptable delivery system or a carrier base composition.

WO02/15875 (P&G) discloses a skin care composition containing an oil-soluble film-forming polymer and an oil-soluble adhesive elastomeric polymer and to methods of using such compositions to regulate the appearance of skin and/or hair.

EP2942048 A1 (Johnson & Johnson, 2015) A discloses a topical composition comprising a substituted resorcinol and a solid powder having a particle size distribution width of at least 15 microns. 4-hexyl resorcinol can lose efficacy or activity when combined with many powders used in topical compositions. The activity of substituted resorcinols can be advantageously maintained when they are used in conjunction with powders possessing the particle size distribution.

The above patent publications do not disclose the inventive combination of the present invention i.e. the skin care actives, the film forming polymers and the specific whitening particles incorporated in a water insoluble substrate for topical application to give the desired benefits.

It is thus an object of the present invention to provide for a mask composition that delivers instant lightening benefits while ensuring efficient delivery of skin care actives.

## Summary of the invention

According to the first aspect of the present invention there is provided a cosmetic mask comprising a water insoluble porous substrate impregnated with

(i) a skin care active selected from retinol, retinyl esters, resorcinol, vitamin B3, allantoin, ubiquinone, conjugated linoleic acid, 12-hydroxystearic acid or derivatives of resorcinol and vitamin B3, wherein said resorcinol derivative is a 4-substituted resorcinol and wherein said vitamin B3 derivative include niacin, nicotinic acid, and niacinamide;
(ii) a skin whitening particle selected from polymeric beads, boron nitride or mica; and
(iii) a film-forming polymer.

According to another aspect of the present invention there is provided a method of lightening skin comprising the steps of

(i) applying the cosmetic mask of the first aspect on to the preferred external skin surface for at least one minute;
(ii) removing the cosmetic mask from said surface; and
(iii) rinsing said surface with water.

## Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are by weight of the entire cosmetic mask including the water insoluble substrate unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Cosmetic Mask" as used herein, is meant to include a mask for topical application to the skin of mammals, especially humans. The mask, as per this invention, is one that is applied to the desired skin surface and left on for a period of time (say from 1 minute to 12 hours) after which it is removed followed preferably by rinsing the skin surface with water. The mask as per the present invention is primarily intended for effective delivery of the skin care active and for skin lightening purposes but may also be formulated into a product which is applied to a human body for improving the appearance, cleansing, odor control or general aesthetics. "Skin" as used herein is meant to include skin on the face and body (e.g., neck, chest, back, arms, underarms, hands, legs, buttocks and scalp) and especially to the sun exposed parts thereof e.g the face, neck or even parts that are known to darken without sun exposure like the underarm. The mask as per the present invention is especially intended for lightening the face of an individual.

The invention provides for a cosmetic mask comprising a water insoluble porous substrate; a skin care composition impregnated in the substrate; wherein the skin care composition comprises a skin care active; a whitening particle and a film forming polymer.

The water insoluble porous substrate for impregnating with the skin care composition is selected from paper, polymeric web, fabric, or composites/mixtures thereof. The porous substrate is preferably paper or fabric. The cosmetic mask preferably comprises 1 to 10%, preferably 2 to 5%, more preferably 3 to 4% by weight of the porous substrate. The porous substrate is preferably configured as discrete sheets, a bundle of such sheets, preferably packed together as a bunch for sale to the consumer. The area of each such sheet is preferably from 100 to 1000 cm$^2$, more preferably 300 to 500 cm$^2$, further more preferably 350 to 450 cm$^2$. The porous substrate in a further preferred aspect is configured in the shape that can be conveniently applied on the face of an individual with holes for the nose, mouth and eyes.

The skin care active for delivery to the skin, as per the present invention is selected from retinol, retinyl esters, resorcinol, vitamin B3, allantoin, ubiquinone, conjugated linoleic acid, 12-hydroxystearic acid or derivatives of resorcinol and vitamin B3, wherein said resorcinol derivative is a 4-substituted resorcinol and wherein said vitamin B3 derivative include niacin, nicotinic acid, and niacinamide.

The term "retinol" includes the following isomers of retinol: all-trans-retinol, 13-cis-retinol, 11-cis-retinol, 9-cis-retinol, 3,4-didehydro-retinol. Preferred isomers are all-trans-retinol, 13-cis-retinol, 3,4-didehydro-retinol, 9-cis-retinol. Most preferred is all-trans-retinol, due to its wide commercial availability.

Retinyl ester is an ester of retinol. Retinyl esters suitable for use in the present invention are $C_1$ -$C_{30}$ esters of retinol, preferably $C_2$ -$C_{20}$ esters, and most preferably $C_2$, $C_3$, and $C_{16}$ esters because they are more commonly available. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl laurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, retinyl oleate, retinyl lactate, retinyl glycolate, retinyl hydroxy caprylate, retinyl hydroxy laurate, refinyl tartarate. The preferred ester for use in the present invention is selected from retinyl palmitate, retinyl acetate and retinyl propionate, because these are efficacious while being most commercially available and therefore the cheapest.

Resorcinols and its derivatives have a wide variety of applications including use in cosmetic products. Resorcinols are reported to have antioxidant, sun-protective, anti-acne, antiseptic and other therapeutic properties. In particular, 4-alkyl resorcinol is reported to have skin-beautifying effect and skin lightening benefits. Especially preferred resorcinol for use in the present invention are 4-substituted resorcinol. Most preferred 4-substituted resorcinol are 4-alkyl resorcinol including 4-ethyl resorcinol, 4-hexyl resorcinol, 4-butyl resorcinol, or 4-phenethyl resorcinol.

Vitamin B3 compounds have been known as skin lightening agents for a long time. They have also been reported to have various other properties beneficial to the skin. Vitamin B3 compound or its derivative include niacin, nicotinic acid, and niacinamide most preferred being niacinamide.

Conjugated linoleic acid (CLA) has been known for the effective treatment and prevention of normal skin conditions due to aging or exposure to light and solar radiation, such as wrinkles, lines, sagging, hyperpigmentation and age spots. Conjugated linoleic acid comprises a group of positional and geometric isomers of linoleic acid in which various configurations of cis and trans double bonds at positions (6, 8), (7, 9), (8, 10), (9, 11), (10, 12) or (11, 13) are possible. Each positional isomer can have 4 geometrical isomers. For example in the case of the (6,8) isomer, there can be cis-6-cis-8, cis-6-trans-8, trans-6-cis-8 and trans-6-trans-8 geometrical isomers. Similarly there can be 4 geometrical isomers for the remaining 5 positional isomers. Thus twenty- four different isomers of the CLA can exist. CLA is different from "linoleic acid". Linoleic acid is a straight chain fatty acid, chemically referred to as cis-9, cis-12-octadecadienoic acid. In the case of linoleic acid the two double bonds are not in conjugation.

Ubiquinone is also known as Coenzyme Q10, it is a 1,4-benzoquinone, where Q refers to the quinone chemical group, and 10 refers to the number of isoprenyl chemical subunits in its tail. It inhibits both the initiation and the propagation of lipid and protein oxidation. It also regenerates other antioxidants such as vitamin E. In cosmetic product, ubiquinone is used as antioxidant.

Allantoin is called 5-ureidohydantoin or glyoxyldiureide. It is a diureide of glyoxylic acid. It is frequently present in lipsticks, cosmetic lotions and creams, and used as antioxidant due to the oxidation of uric acid by purine catabolism.

12-hydroxystearic acid (generally referred to as "12-HSA") is reported to have a wide variety of beneficial cosmetic effects on skin, e.g. it is a known as a PPAR-alpha (peroxisome proliferator activated receptors sub-type alpha) activator, skin lightening agent, and a sebum secretion inhibitor. 12-HSA has also traditionally been used as gelling agent e.g. in lipsticks and anti-perspirant compositions. 12-HSA has also been used to increase viscosity of cosmetic compositions.

It is preferred that the skin care active for inclusion in the cosmetic mask of the invention is selected from resorcinol, vitamin B3, conjugated linoleic acid, 12-hydroxystearic acid or derivatives thereof. It is preferred that the skin care active is present in 0.001 to 5% by weight of the cosmetic mask.

"Film-forming polymer" as used herein refers to a polymer which is capable of forming cohesive and continuous covering over the hair and/or skin when applied to their surface. Contact angle, as used herein, means the angle at which a water/vapor interface meets a solid surface at a temperature of 25°C. Such an angle may be measured with a goniometer or other water droplet shape analysis systems with water droplet of 5 μl and at 25°C. The requirement for film-forming polymer, as per the present invention is that the film-forming polymer is suitable to be employed in a cosmetic composition. The film-forming polymer, for use in the present invention, preferably has a contact angle of at least 85°. Not wishing to be bound by theory, the inventors believe that such a compact and continuous film covers the skin surface and helps to reduce the loss of the whitening particles during washing or later through abrasion. The film forming polymer is generally water insoluble and is distinct from water soluble polymers like cross-linked polyacrylic acid sold as Carbopol which are commonly used in cosmetic product as thickeners.

The film forming polymer as per the invention is selected from an acrylate polymer, a methacrylate polymer, a urethane polymer or co-polymers thereof. Especially preferred are acrylate co-polymer and a urethane-acrylic co-polymer. Commercially available film forming polymers which may be used in the present invention are KOBO-50N available from Kobo and Hybridur 875 from Air Products. The film forming polymer is included in 1 to 15%, preferably in 1 to 8 %, more preferably in 2 to 6% by weight of the cosmetic mask.

A whitening particle is included in the cosmetic composition for impregnation in the cosmetic mask of the invention. The whitening particle is selected from one of a polymer bead, boron nitride or mica. When a polymer bead is included, it is preferably a hollow sphere. Suitable hollow spheres are those made of a styrene/ acrylate co-polymer, polyurethane, or polyethylene. Most preferred polymeric bead is a hollow sphere made of a styrene/ acrylate co-polymer. Polymer beads which may be included in the composition of the invention preferably have a particle size in the range of 80 to 700 nm. Commercially available hollow sphere polymer beads which may be used in the composition of the invention are Sunspheres™ (from Dow).

Boron nitride generally has a hexagonal crystal structure of BN stacked over each other. Different crystal structures gives rise to different types of boron nitride namely: graphitic, turbostratic and quasigraphitic. Graphitic BN is the purest BN crystals where all the hexagonal BN crystals are aligned parallel with each other. Turbostratic BN has oxygen impurity in the BN crystal lattice. Some of the nitrogen (typically <2%) are replaced with oxygen. Presence of Oxygen distorts the crystal stacking and the planes of BN are not exactly parallel to each other like in case of graphitic BN. Use of these turbostratic BN crystals when used in a composition for application to the skin provides a matte effect as there is less specular reflection from them. Quasi graphitic BN is somewhere in between these two morphologies.

Mica is a naturally occurring substance generally found of igneous origin. It belongs to the group of sheet silicate or phyllosilicate minerals. They generally have a nearly perfect basal cleavage. They are generally monoclinic crystals, with a tendency towards pseudohexagonal crystals. Mica may be selected from muscovite, phlogopite, tiotite, sericite, lepidolite, paragonite and artificial or synthetic mica having a fluorine atom substituted for the hydroxyl group of natural mica as well as baked or calcined products thereof. These mica may be used alone or in any mixture thereof.

The whitening particle, as per the present invention, is preferably present in 0.1 to 5.0 % by weight of the cosmetic mask. It is preferably present as particles having an average particle size from 80 nm to 20 microns.

The cosmetic mask of the invention may additionally comprise a surfactant. A surfactant of the anionic or non-ionic form is especially preferred. Suitable surfactants include fatty alcohols and esters of fatty acids. Especially preferred and widely and inexpensively available surfactants of the above classes which may be included in the compositions of the present invention are cetearyl alcohol, glyceryl monostearate and PEG-20 stearate and (PEG-60 stearate). Such surfactants are preferably included in 0.1% to 20%, more preferably included in 0.1-8% by weight of the cosmetic mask.

The cosmetic mask preferably additionally comprises water. Water may be present in 20 to 96%, preferably 40 to 90%, more preferably 50 to 80% by weight of the cosmetic mask.

The cosmetic mask of the present invention can comprise a wide range of other optional components. The CTFA Personal care Ingredient Handbook, Second Edition, 1992, which is incorporated by reference herein in its entirety, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, opacifying agents, conditioners, exfoliating agents, pH adjusters, preservatives, natural extracts, essential oils, skin sensates, skin soothing agents, and skin healing agents.

According to another aspect of the present invention there is provided a method of lightening skin comprising the steps of applying the cosmetic mask of the invention on to the preferred external skin surface for at least one minute and removing the mask from the surface. Preferably thereafter the surface is rinsed with water. The method is preferably non-therapeutic. The mask is preferably left on to the desired skin surface from 1 minute to 12 hours, preferably 5 minutes to 4 hours, more preferably 10 minutes to an hour before it may be removed. The desired skin surface is preferably rinsed with water from 0 minutes to 2 hours, preferably 0 minute to 1 hours, preferably 5 minutes to an hour after removing the cosmetic mask.

The invention will now be illustrated with the help of the following non-limiting examples.

**Examples**

Examples 1-2: Effect of using polymeric beads instead of a conventional titanium dioxide whitening particle in a mask comprising a biological active

The following compositions were impregnated in a non-woven fabric of 0.062 grams weight.

Table -1

| Example | 1 | 2 |
|---|---|---|
| Hexyl resorcinol, wt% | 0.01 | 0.01 |
| Sodium sulfite, wt% | 0.005 | 0.005 |
| Whitening particle | Titanium dioxide[1] | Sunsphere[2] |
| Whitening particle, wt% | 0.4 | 4.0 |
| [1] TiO$_2$ used was Kowet available from Sensient with a particle size of 500 nm to 2 microns<br>[2] Sunsphere is a hollow microsphere polymer made of styrene/ acrylate co-polymer with an average particle size of 400 nm sourced from Dow Chemicals. | | |

The mask prepared above was stored in a hot chamber at 45 °C for one month to study the stability of the samples. The samples at the end of one month were studied visually. The sample of Example 1 had a distinct yellowish tinge which is not liked by the consumer while the sample of Example 2 was still whitish. To quantify the colour, the samples were measured for colour using Digieye colour measurement and imaging system by VerVide.

The Δ value is the difference between the final and initial values. L is an indication of whiteness (higher the L value the whiter the colour). b is an indication of the yellowness (higher the b value the more yellow is the material) and E is a consolidated measure of the increase in colour and is calculated using

$$\Delta E = \sqrt{\Delta L^2 + \Delta a^2 + \Delta b^2}$$

The relevant colour differential values are given in the table below:

Table - 2

| Example | 1 | 2 |
|---|---|---|
| ΔL | -3.3 | -2.44 |
| Δb | 5.04 | 2.64 |
| ΔE | 6.02 | 4.04 |

The data in Table 2 above indicates that when a biological active is used in a mask composition it is preferable to use a whitening particle like polymeric beads as compared to an inorganic material like titanium dioxide. The yellowing is seen when even 0.4% TiO$_2$ is used while no yellowing is observed with as high as 4% sunspheres.

Example 4-5: Effect of using film forming polymer

The following compositions were prepared and impregnated in a non-woven fabric.

Table-3

| Example | 3 | 4 |
|---|---|---|
| Glycerin, wt% | 5.0 | 5.0 |
| Butylene glycol, wt% | 2.0 | 2.0 |
| PEG/PPG Copolymer, wt% | 1.0 | 1.0 |

(continued)

| Example | 3 | 4 |
|---|---|---|
| Sunsphere, wt% | 2.0 | 2.0 |
| Hexyl resorcinol, wt% | 0.005 | 0.005 |
| Film forming polymer Kobo 50N, wt% | - | 4.0 |
| Water | To 100 | To 100 |

The procedure used to measure the whitening efficacy was as follows:
0.6 gram of the above composition was impregnated in a non-woven fabric of 3 cm diameter and 0.062 gram weight. After about 5 minutes of applying the composition on the fabric, it was placed on a skin surrogate substrate (30#BSP) for 15 minutes. After this the fabric was removed and the substrate was rinsed with water for 15 seconds. The substrate was air dried using pressurised air. The L*a*b* values of the substrate was then measured using Digieye colour measurement and imaging system by VerVide.

The data in Table 4 below indicates the change in colour (ΔL) for the two samples.

Table - 4

| Example | 3 | 4 |
|---|---|---|
| ΔL | 0.19 | 1.02 |

The data in Table -4 above indicates that mask as per the invention (Example 4) provides for a better skin lightening as compared to a mask outside the invention (Example -3).

The colour was measured using a slightly different washing protocol as below. 0.6 gram of the above composition was impregnated in a non-woven fabric of 3 cm diameter and 0.062 gram weight. After about 5 minutes of applying the composition on the fabric, it was placed on a skin surrogate substrate (30#BSP) for 15 minutes. After this the fabric was removed and the substrate was allowed to dry at 25 °C for one hour. It was then rinsed with water for 15 seconds. The substrate was then air dried using pressurised air. The L*a*b* values of the substrate was then measured using Digieye colour measurement and imaging system by VerVide.

The data generate using the above procedure is given below in Table -5:

Table - 5

| Example | 3 | 4 |
|---|---|---|
| ΔL | 2.26 | 4.61 |

The data in Table -5 above indicates that mask as per the invention (Example 4) provides for a better skin lightening as compared to a mask outside the invention (Example -3) even with a different usage protocol.

**Claims**

1. A cosmetic mask comprising a water insoluble porous substrate impregnated with

   (i) a skin care active selected from retinol, retinyl esters, resorcinol, vitamin B3, allantoin, ubiquinone, conjugated linoleic acid, 12-hydroxystearic acid or derivatives of resorcinol and vitamin B3, wherein said resorcinol derivative is a 4-substituted resorcinol, and wherein said vitamin B3 derivative include niacin, nicotinic acid, and niacinamide;
   (ii) a skin whitening particle selected from polymeric beads, boron nitride, or mica; and
   (iii) a film-forming polymer.

2. A cosmetic mask as claimed in claim 1 wherein said water insoluble porous substrate is selected from paper, polymeric web, fabric, or composites/mixtures thereof.

3. A cosmetic mask as claimed in claim 1 or 2 wherein said polymeric bead is a hollow sphere made of a styrene/ acrylate co-polymer.

4. A cosmetic mask as claimed in any one of the preceding claims wherein said skin care active is selected from resorcinol, vitamin B3, conjugated linoleic acid, 12-hydroxystearic acid or derivatives thereof.

5. A cosmetic mask as claimed in any one of the preceding claims wherein said 4-substituted resorcinol includes 4-ethyl resorcinol, 4-hexyl resorcinol, 4-butyl resorcinol or 4-phenethyl resorcinol.

6. A cosmetic mask as claimed in any one of the preceding claims wherein said skin care active is present in 0.001 to 5% by weight of the cosmetic mask.

7. A cosmetic mask as claimed in any one of the preceding claims wherein the whitening particle is present in 0.1 to 5.0 % by weight of the cosmetic mask.

8. A cosmetic mask as claimed in any one of the preceding claims wherein the whitening particle has a particle size from 80 nm to 20 microns.

9. A cosmetic mask as claimed in any one of the preceding claims wherein said film forming polymer is selected from an acrylate polymer, a methacrylate polymer, a urethane polymer or co-polymers thereof.

10. A cosmetic mask as claimed in claim 9 wherein said polymer is an acrylate copolymer or a polyurethane-methylmethacrylate co-polymer.

11. A cosmetic mask as claimed in any one of the preceding claims comprising 0.1 to 15% film forming polymer by weight of the cosmetic mask.

12. A cosmetic mask as claimed in any one of the preceding claims comprising 80 to 95 % water by weight of the mask.

13. A method of lightening skin comprising the steps of :

(i) applying the cosmetic mask as claimed in any one of the preceding claims on to the preferred external skin surface for at least one minute;
(ii) removing the cosmetic mask from said surface; and
(iii) rinsing said surface with water.

14. A method as claimed in claim 13 wherein said step (iii) is carried out from 0 to 60 minutes after step (ii).


**Patentansprüche**

1. Kosmetische Maske, umfassend ein wasserunlösliches poröses Substrat, imprägniert mit

(i) einem Hautpflegewirkstoff, ausgewählt aus Retinol, Retinylestern, Resorcin, Vitamin B3, Allantoin, Ubichinon, konjugierter Linolsäure, 12-Hydroxystearinsäure oder Derivaten von Resorcin und Vitamin B3, wobei das Resorcin-Derivat ein 4-substituiertes Resorcin ist und wobei das Vitamin B3-Derivat Niacin, Nicotinsäure und Niacinamid einschließt;
(ii) einem Hautaufhellerpartikel, ausgewählt aus polymeren Kügelchen, Bornitrid oder Glimmer, und
(iii) einem filmbildenden Polymer.

2. Kosmetische Maske, wie im Anspruch 1 beansprucht, wobei das wasserunlösliche poröse Substrat aus Papier, Polymergewebe, textilem Material oder Kompositmaterialien/Mischungen davon ausgewählt ist.

3. Kosmetische Maske, wie im Anspruch 1 oder 2 beansprucht, wobei das polymere Kügelchen eine Hohlkugel ist, die aus Styrol/Acrylat-Copolymer hergestellt ist.

4. Kosmetische Maske, wie in irgendeinem der vorhergehenden Ansprüche beansprucht, wobei der Hautpflegewirkstoff aus Resorcin, Vitamin B3, konjugierter Linolsäure, 12-Hydroxystearinsäure oder Derivaten davon ausgewählt ist.

**5.** Kosmetische Maske, wie in irgendeinem der vorhergehenden Ansprüche beansprucht, wobei das 4-substituierte Resorcin 4-Ethylresorcin, 4-Hexylresorcin, 4-Butylresorcin oder 4-Phenethylresorcin einschließt.

**6.** Kosmetische Maske, wie in irgendeinem der vorhergehenden Ansprüche beansprucht, wobei der Hautpflegewirkstoff mit 0,001 bis 5%, bezogen auf das Gewicht der kosmetischen Maske, vorliegt.

**7.** Kosmetische Maske, wie in irgendeinem der vorhergehenden Ansprüche beansprucht, wobei das Aufhellerpartikel mit 0,1 bis 5,0%, bezogen auf das Gewicht der kosmetischen Maske, vorliegt.

**8.** Kosmetische Maske, wie in irgendeinem der vorhergehenden Ansprüche beansprucht, wobei das Aufhellerpartikel eine Partikelgröße von 80 nm bis 20 Mikrometer aufweist.

**9.** Kosmetische Maske, wie in irgendeinem der vorhergehenden Ansprüche beansprucht, wobei das filmbildende Polymer aus einem Acrylat-Polymer, einem Methacrylat-Polymer, einem Urethan-Polymer oder Co-Polymeren davon ausgewählt ist.

**10.** Kosmetische Maske, wie im Anspruch 9 beansprucht, wobei das Polymer ein Acrylat-Copolymer oder ein Polyurethan/Methylmethacrylat-Copolymer ist.

**11.** Kosmetische Maske, wie in irgendeinem der vorhergehenden Ansprüche beansprucht, umfassend 0,1 bis 15% filmbildendes Polymer, bezogen auf das Gewicht der kosmetischen Maske.

**12.** Kosmetische Maske, wie in irgendeinem der vorhergehenden Ansprüche beansprucht, umfassend 80 bis 95% Wasser, bezogen auf das Gewicht der Maske.

**13.** Verfahren zum Aufhellen von Haut, umfassend die Schritte:

(i) Auftragen der kosmetischen Maske, wie in irgendeinem der vorhergehenden Ansprüche beansprucht, auf die bevorzugte externe Oberfläche der Haut für mindestens eine Minute;
(ii) Entfernen der kosmetischen Maske von der Oberfläche und
(iii) Abspülen der Oberfläche mit Wasser.

**14.** Verfahren, wie im Anspruch 13 beansprucht, wobei der Schritt (iii) 0 bis 60 Minuten nach dem Schritt (ii) durchgeführt wird.

**Revendications**

**1.** Masque cosmétique comprenant un substrat poreux insoluble dans l'eau imprégné avec

(i) un actif pour le soin de la peau choisi parmi le rétinol, des esters rétinyliques, le résorcinol, la vitamine B3, l'allantoïne, l'ubiquinone, l'acide linoléique conjugué, l'acide 12-hydroxystéarique et des dérivés de résorcinol et de vitamine B3, dans lequel ledit dérivé de résorcinol est un résorcinol 4-substitué, et dans lequel ledit dérivé de vitamine B3 comprend la niacine, l'acide nicotinique, et le niacinamide ;
(ii) une particule blanchissant la peau choisie parmi des billes polymères, le nitrure de bore, ou le mica ; et
(iii) un polymère formant un film.

**2.** Masque cosmétique selon la revendication 1, dans lequel ledit substrat poreux insoluble dans l'eau est choisi parmi le papier, une bande polymère, un textile, ou des composites/mélanges de ceux-ci.

**3.** Masque cosmétique selon la revendication 1 ou 2, dans lequel ladite bille polymère est une sphère creuse constituée d'un copolymère de styrène/acrylate.

**4.** Masque cosmétique selon l'une quelconque des revendications précédentes, dans lequel ledit actif pour le soin de la peau est choisi parmi le résorcinol, la vitamine B3, l'acide linoléique conjugué, l'acide 12-hydroxystéarique ou des dérivés de ceux-ci.

**5.** Masque cosmétique selon l'une quelconque des revendications précédentes, dans lequel ledit résorcinol 4-substitué

9

comprend le 4-éthylrésorcinol, 4-hexylrésorcinol, 4-butylrésorcinol ou 4-phénéthylrésorcinol.

6. Masque cosmétique selon l'une quelconque des revendications précédentes, dans lequel ledit actif pour le soin de la peau est présent dans de 0,001 à 5 % en masse du masque cosmétique.

7. Masque cosmétique selon l'une quelconque des revendications précédentes, dans lequel la particule blanchissante est présente dans de 0,1 à 5,0 % en masse du masque cosmétique.

8. Masque cosmétique selon l'une quelconque des revendications précédentes, dans lequel la particule blanchissante présente une taille de particule de 80 nm à 20 microns.

9. Masque cosmétique selon l'une quelconque des revendications précédentes, dans lequel ledit polymère formant un film est choisi parmi un polymère d'acrylate, un polymère de méthacrylate, un polymère d'uréthane ou des co-polymères de ceux-ci.

10. Masque cosmétique selon la revendication 9, dans lequel ledit polymère est un copolymère d'acrylate ou un co-polymère de polyuréthane-méthacrylate de méthyle.

11. Masque cosmétique selon l'une quelconque des revendications précédentes comprenant de 0,1 à 15 % de polymère formant un film en masse du masque cosmétique.

12. Masque cosmétique selon l'une quelconque des revendications précédentes comprenant de 80 à 95 % d'eau en masse du masque.

13. Procédé d'éclaircissement de la peau comprenant les étapes de :

(i) application du masque cosmétique selon l'une quelconque des revendications précédentes sur la surface de peau externe préférée pendant au moins une minute ;
(ii) élimination du masque cosmétique de ladite surface de peau ; et
(iii) rinçage de ladite surface avec de l'eau.

14. Procédé selon la revendication 13, dans lequel ladite étape (iii) est réalisée pendant de 0 à 60 minutes après l'étape (ii).

**REFERENCES CITED IN THE DESCRIPTION**

**Patent documents cited in the description**

- US 2004219124 A
- WO 0215875 P

- EP 2942048 A1, Johnson & Johnson